Europäisches Patentamt

European Patent Office

Office européen des brevets

(11) Veröffentlichungsnummer : **0 445 158 B1**

## (12) EUROPÄISCHE PATENTSCHRIFT

(45) Veröffentlichungstag der Patentschrift :
04.08.93 Patentblatt 93/31

(51) Int. Cl.⁵ : **A61K 31/505,** A61K 9/18,
A61K 47/32, A61K 47/34

(21) Anmeldenummer : 89912952.2

(22) Anmeldetag : 24.11.89

(86) Internationale Anmeldenummer :
PCT/EP89/01425

(87) Internationale Veröffentlichungsnummer :
WO 90/06115 14.06.90 Gazette 90/14

(54) ZUBEREITUNGEN DES OXIPURINOLS UND/ODER SEINER ALKALI- ODER ERDALKALISALZE.

(30) Priorität : 25.11.88 DE 3839825

(43) Veröffentlichungstag der Anmeldung :
11.09.91 Patentblatt 91/37

(45) Bekanntmachung des Hinweises auf die
Patenterteilung :
04.08.93 Patentblatt 93/31

(84) Benannte Vertragsstaaten :
AT BE CH DE ES FR GB IT LI LU NL SE

(56) Entgegenhaltungen :
DE-A- 2 546 371
GB-A- 975 850
CHEMICAL ABSTRACTS, vol. 83, no. 2, 14 July
1975, Columbus, OH (US); J.H.COLLETT et al.,
p. 340, no. 15544t
JOURNAL OF PHARMACEUTICAL SCIENCES,
vol. 60, no. 9, September 1971; Win Loung
Chiou et al., pp. 1281-1302

(73) Patentinhaber : HENNING BERLIN GmbH
CHEMIE- UND PHARMAWERK
Komturstrasse 58-62
W-1000 Berlin 42 (DE)

(72) Erfinder : LAHR, Wolfgang
Laubacher Strasse 1
W-1000 Berlin 41 (DE)
Erfinder : WEICKGENANNT, Guido
Beerenstrasse 33
W-1000 Berlin 37 (DE)

(74) Vertreter : Werner, Hans-Karsten et al
Patentanwälte von Kreisler-Selting-Werner
Schönwald-Fues-Dallmeyer Deichmannhaus
W-5000 Köln 1 (DE)

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Oxipurinol (4,6-Dihydroxypyrazolo<3,4-d>pyrimidin) stellt den aktiven Hauptmetaboliten des für die Gichttherapie bekannten Allopurinols dar. Oxipurinol ist in Wasser und Verdauungssäften schwer löslich, weshalb es bislang wegen der damit verbundenen unzureichenden Resorbierbarkeit praktisch in der Therapie keinen Eingang gefunden hat.

Aus der DE-OS 37 07 999 ist die Verwendung von Oxipurinol zur Verminderung von Zellschäden, und zwar in Form einer injektablen Darreichungsform, bekannt. Die orale Anwendung dieser Präparationen nach dem Stand der Technik ist nicht erwähnt und wegen der bei diesen Formen vorherrschenden pH-Werten von bis zu pH 12 wegen der zu erwartenden Verätzungen nach oraler Einnahme auch nicht praktikabel.

Alkali- und Erdalkalisalze des Oxipurinols weisen eine verbesserte Löslichkeit gegenüber der Säure auf. Wäßrige Lösungen von Oxipurinol-Natrium-Monohydrat weisen beispielsweise einen pH-Wert von 9,5 auf und lassen Konzentrationen bei 25°C von etwa 1,5% zu. Derartige pH-Werte sind jedoch unphysiologisch.

Unter physiologischen Verhältnissen, d.h. bei pH-Werten von etwa 1,0 bis 7,5, wie sie im menschlichen Verdauungstrakt vorherrschen, ist die Löslichkeit nach wie vor unzureichend. Insbesondere ist die Lösungsgeschwindigkeit des Oxipurinols und seiner Salze gering. So wurde die Löslichkeit des Oxipurinol-Natrium-Monohydrats in 0,1 N Salzsäure, was in etwa der Acidität des nüchternen Magens entspricht, mit etwa 43 mg/l nach 5 Minuten (etwa 0,004%) bzw. 142 mg/l (etwa 0,014%) nach 60 Minuten bestimmt.

Unter Lösungsgeschwindigkeit wird dabei im Sinne der Erfindung die Masse Substanz verstanden, die in einer bestimmten Zeit gelöst wird. Unter Löslichkeit wird hingegen die Masse Substanz verstanden, die in einer Masse bzw. einem bestimmten Volumen Lösungsmittel klar gelöst ist.

Die Löslichkeit und die Lösungsgeschwindigkeit eines Arzneimittelstoffes sind für dessen Anflutung im systemischen Kreislauf und damit für das Ausmaß der Bioverfügbarkeit geschwindigkeitsbestimmend.

Der Erfindung lag somit die Aufgabe zugrunde, die Löslichkeit und die Lösungsgeschwindigkeit des Oxipurinols zu verbessern.

Diese Aufgabe kann überraschend einfach gelöst werden durch Zubereitungen des Oxipurinols und/oder seiner Alkali- oder Erdalkalisalze, die dadurch gekennzeichnet sind, daß sie den Wirkstoff in nicht kristalliner Form in Form einer sogenannten "festen Dispersion" mit pharmakologisch unbedenklichen Hilfsstoffen im Verhältnis 1:0,2 bis 1:10, vorzugsweise im Verhältnis 1:1 bis 1:4 enthalten.

Es werden so Zubereitungen erhalten, die dadurch gekennzeichnet sind, daß das darin enthaltene Oxipurinol eine Lösungsgeschwindigkeit bei einem pH-Wert zwischen 1,0 und 7,5 von mehr als 100 mg/l pro 5 Minuten besitzt.

Besonders bevorzugt sind Zubereitungen, die dadurch gekennzeichnet sind, daß die terminale (vorübergehende) Löslichkeit des darin enthaltenen Oxipurinols über der Sättigungskonzentration liegt und länger als eine Stunde aufrechterhalten wird.

Diese Zubereitungen können gewünschtenfalls auch weitere Wirkstoffe enthalten oder mit diesen vermischt vorliegen.

Die Herstellung der erfindungsgemäßen Zubereitungen erfolgt dadurch, daß Oxipurinol und/oder seine Alkalioder Erdalkalisalze zusammen mit den Hilfsstoffen gegebenenfalls unter Zusatz eines Lösungsmittels gelöst werden oder in einer Hilfsstoffschmelze aufgelöst werden und die erhaltenen Schmelzen oder Lösungen abgekühlt und/oder eingetrocknet werden.

Als Lösungsmittel wird vorzugsweise Wasser verwendet.

Als pharmakologisch unbedenkliche Hilfsstoffe kommen prinzipiell alle Hilfsstoffe in Frage, die entweder aufgeschmolzen oder mit Hilfe eines Lösungsmittel gelöst werden können, wobei solche Hilfsstoffe bevorzugt sind, in deren Schmelzen oder Lösungen auch Oxipurinol und/oder seine Alkali- oder Erdalkalisalze in erheblichem Umfang gelöst werden können. Werden derartige Schmelzen oder Lösungen rasch abgekühlt und/oder eingetrocknet, so entstehen Feststoffdispersionen oder sogenannte "feste Lösungen", in denen das Oxipurinol und/oder seine Alkali- oder Erdalkalisalze in nicht kristalliner Form vorliegen.

Die Herstellung der erfindungsgemäßen Zubereitungen erfolgt beispielsweise dadurch, daß der Wirkstoff und die Hilfsstoffe miteinander aufgeschmolzen und durch Ausgießen zu Platten, Abtropfen auf gekühlte Unterlagen zu Perlen, Eingießen in vorgezogene Blisternäpfe oder Sprüherstarrung abgekühlt werden. Diese Schmelzen können gegebenenfalls auch direkt in Kapseln eindosiert werden.

Besonders einfach und schonend erfolgt die Herstellung durch Zugabe eines Lösungsmittels. Als Lösungsmittel kommen prinzipiell alle leicht entfernbaren organischen Lösungsmittel und Lösungsmittelgemische wie Alkohole und Alkohol-Wasser-Gemische in Frage. Ökologisch und ökonomisch besonders bevorzugt ist Wasser als Lösungsmittel.

Die Herstellung der Lösungen kann beispielsweise dadurch erfolgen, daß zunächst nur der Wirkstoff in dem Lösungsmittel vorgelöst wird und dann eine Lösung der Hilfsstoffe zugemischt wird. Es ist auch möglich,

EP 0 445 158 B1

den Wirkstoff nachträglich in eine Lösung des Hilfsmittels einzubringen. Das Entfernen des Lösungsmittels erfolgt anschließend durch Verdunsten, Verdampfen im Vakuum, durch Sprühtrocknung oder Gefriertrocknung.

Bevorzugte Hilfsstoffe zur Herstellung der Feststoffdispersionen sind Polyethylenglykole mit mittleren Molekulargewichten von 200 is etwa 35.000, Polyvinylpyrrolidon (z.B. Kollidon®17, 25, 30, 90), Polyvinylacetat, Mischpolymerisate aus Polyvinylpyrrolidon/Polyvinylacetat (z.B. Kollidon® VA 64), Polyvinylalkohole unterschiedlichster Verseifungsgrade, Cellulosederivate wie Natriumcarboxymethylcellulose, Hydroxyethylcellulose, Hydroxypropylmethylcellulose, Acrylsäurederivate wie Polyacrylsäure (z.B. Carbopol®), wobei die Hilfsstoffe allein oder als Mischung eingesetzt werden. Besonders bevorzugte Hilfsstoffe sind Polyethylenglykole und Polyvinylpyrrolidone. Gegebenenfalls werden zusätzlich noch Tenside oder Netzmittel zugesetzt. Das Verhältnis Oxipurinol oder eines seiner Salze zu den verwendeten Hilfsstoffen beträgt 1:0,2 bis 1:10, wobei Verhältnisse von über 1:0,8 besonders bevorzugt sind, insbesondere wenn eine Feststoffdispersion erhalten werden soll, die frei von restlichen kristallinen Anteilen ist. Besonders bevorzugt werden somit Verhältnisse von 1:1 bis 1:4, um Arzneiformen herzustellen, die von der Masse oder den Abmessungen her die Verabreichung erleichtern, da besonders große Arzneiformen nicht die gewünschte Patientenkomplience gewährleisten. Die getrockneten oder erstarrten Feststoffdispersionen können entweder direkt dosisgerecht für die orale Verabreichung, beispielsweise in Form von Kapseln, abgeteilt werden oder zusammen mit weiteren Hilfsstoffen wie Füllstoffen, Zerfallsförderern, Netzmitteln, Gleit- und Schmiermitteln zu Tabletten verpreßt werden. Gegebenenfalls erhalten derartige Tabletten einen Überzug aus beispielsweise Acrylsäure- oder Cellulosederivaten, dem Pigmente und Farbstoffe zugemischt werden können. Werden bei hoher Dosierung große Arzneiformen hergestellt, die die Einnahme erschweren könnten, wie beispielsweise Tabletten, so erhalten diese eine oder mehrere Teilungskerben, so daß Bruchstücke der Arzneiform oder Teildosen verabreicht werden können.

Selbstverständlich lassen sich die Feststoffdispersionen aus Oxipurinol und/oder seinen Alkali- oder Erdalkalisalzen auch mit weiteren Wirksubstanzen zu Kombinationspräparaten verarbeiten, beispielsweise mit Benzbromaron. Des weiteren können auch Arzneiformen hergestellt werden, die Oxipurinol bevorzugt in Form einer Mischung aus Feststoffdispersion und kristallinem Wirkstoff enthalten.

Eine weitere Möglichkeit zur Herstellung von Feststoffdispersionen aus Oxipurinol oder seinen Salzen besteht darin, zu den vorbeschriebenen Lösungen bzw. Schmelzen vor dem Trocknen oder Erstarren weitere Hilfsstoffe zuzugeben, die beispielsweise die Tablettierfähigkeit der Feststoffdispersion verbessern oder die Zerfallbarkeit der Arzneiform beschleunigen. Derartige Hilfsstoffe sind solche, die zur Bildung einer Feststoffdispersion keinen oder nur geringen Beitrag leisten, wie beispielsweise mikrokristalline Cellulose, quervernetztes Polyvinylpyrrolidon, Lactose oder Stärke. Nach dem Entfernen des Lösungsmittels oder nach dem Erstarren wird ein Gemisch der Dispersion mit diesen Hilfsstoffen erhalten, welches nach bekannten Verfahren zu den genannten Arzneiformen verarbeitet werden kann.

Feststoffdispersionen der vorbeschriebenen Art oder daraus hergestellte Arzneiformen setzen den Wirkstoff in Kontakt mit natürlichen oder künstlichen Verdauungssäften in einem pH-Bereich von etwa 1,0 bis 7,5, also pH-Bereichwerten, wie sie im menschlichen Verdauungstrakt vorherrschen, rasch frei. Insbesondere ist die Lösungsgeschwindigkeit des Oxipurinols oder eines seiner Salze im stark sauren Bereich, nämlich bei pH-Werten von etwa 1,0 bis 2,0, also Verhältnissen, wie sie im nüchternen Zustand im Magen vorherrschen können, gegenüber den reinen Wirkstoffen um ein Mehrfaches erhöht, wobei diejenige Menge gelöster Wirkstoff als Maß für die Lösungsgeschwindigkeit gilt, die nach 5 Minuten Verweilzeit im Prüfmedium gelöst vorliegt.

Die Löslichkeit eines Feststoffes ist unter normierten Bedingungen eine Stoffkonstante. Für Oxipurinol-Natrium-Monohydrat wurde beispielsweise gefunden, daß die maximale Löslichkeit nach 24 Stunden bei etwa 280 mg/l Lösungsmittel liegt, um nach weiteren 24 Stunden langsam wieder abzufallen. Somit kann die Löslichkeit des Oxipurinol-Natrium-Monohydrats als Stoffkonstante mit etwa 260 mg/l festgelegt werden. Mit der erhöhten Lösungsgeschwindigkeit der erfindungsgemäßen Zubereitungen geht parallel eine erhöhte Löslichkeit einher, die zu übersättigten Lösungen führt. Es war deshalb überraschend festzustellen, daß sich aus den erfindungsgemäßen Zubereitungen eine erhöhte "terminale Löslichkeit" ergibt, die zumindest mehrere Stunden aufrechterhalten wird, um erst dann entsprechend der Löslichkeit als Stoffkonstante wieder bis zum Sättigungsgrad der Lösung abzufallen.

Durch die erhöhte Lösungsgeschwindigkeit und erhöhte "terminale Löslichkeit" des Oxipurinols bzw. seiner Salze kann der Wirkstoff schnell und in hohem Maße resorbiert werden. Er ist entsprechend gut bioverfügbar. Die erfindungsgemäßen Zubereitungen sind demnach bestens geeignet zur Verwendung in Arzneimitteln zur Behandlung der Hyperurikämie bzw. der Gicht.

In den nachfolgenden Beispielen sind die erfindungsgemäßen Zubereitungen, ihre Herstellung und ihre Verwendung näher erläutert.

3

**Beispiel 1**

| | |
|---|---|
| Oxipurinol-Natrium-Monohydrat | 6,0 g |
| Polyethylenglycol (Mittl. Mol.-Gewicht 6000) | 18,0 g |
| Polyvinylpyrrolidon (Kollidon® 25) | 6,0 g |
| Destilliertes Wasser | 96,0 g |

Polyethylenglycol und Kollidon® werden in wasser vorgelöst. Unter Erwärmen wird das Oxipurinol-Salz gelöst. Die praktisch klare Lösung wird im Vakuum zur Trockne eingedampft.

Es entstehen glasige Produkte, die nach mikroskopischer Beurteilung keine Kristalle enthalten.

**Beispiel 2**

| | |
|---|---|
| Oxipurinol-Natrium-Monohydrat | 2,0 g |
| Polyvinylalkohol (Mowiol®, Typ 8-88) | 2,0 g |
| Polyethylenglykol (Mittl. Mol.-Gewicht 6000) | 1,0 g |
| Destilliertes Wasser | 40,0 g |

Nacheinander werden Polyvinylalkohol, Polyethylenglykol und das Oxipurinol-Salz unter Erwärmen in Wasser gelöst. Nach dem Abdampfen des Wassers im Vakuum entsteht ein glasiges, praktisch kristallfreies Produkt.

**Beispiel 3**

| | |
|---|---|
| Oxipurinol-Natrium-Monohydrat | 1,0 g |
| Destilliertes Wasser | 30,0 g |
| | |
| Polyvinylpyrrolidon/Polyvinylacetat-Mischpolymerisat (Kollidon® VA 64) | 1,0 g |
| Polyethylenglykol (Mittl. Mol.-Gewicht 6000) | 1,0 g |

Oxipurinol wird in Wasser unter Erwärmen gelöst. Nach Zugabe von Kollidon® und Polyethylenglykol wird die klare Lösung zur Trockne eingedampft.

**Beispiel 4**

| | |
|---|---|
| Oxipurinol-Natrium-Monohydrat | 5,0 g |
| Destilliertes Wasser | 250,0 g |
| Polyvinylpyrrolidon/Polyvinylacetat-Mischpolymerisat (Kollidon® VA 64) | 2,5 g |
| Polyethylenglykol (Mittl. Mol.-Gewicht 10000) | 2,5 g |

Oxipurinol wird in Wasser unter Erwärmen gelöst. Nach Zugabe von Kollidon® und Polyethylenglykol wird die klare Lösung im Sprühtrockner in einem Heißluftstrom von ca. 150°C getrocknet.

**Beispiel 5**

| | |
|---|---|
| Oxipurinol (freie Säure) | 5,0 g |
| Destilliertes Wasser | 100,0 g |
| Polyvinylpyrrolidon/Polyvinylacetat-Mischpolymerisat (Kollidon® VA 64) | 1,0 g |
| Polyethylenglykol (Mittl. Mol.-Gewicht 6000) | 1,0 g |

Oxipurinol wird in Wasser bei 95°C gelöst. Nach Zugabe von Kollidon® und Polyethylenglykol erfolgt im Vakuum Eindampfen zur Trockne.

**Beispiel 6**

| | |
|---|---|
| Oxipurinol-Natrium-Monohydrat | 0,5 g |
| Destilliertes Wasser | 50,0 g |
| Hydroxypropylcellulose | 2,0 g |

Oxipurinol wird in Wasser bei 80°C gelöst. Die Cellulose wird in der Lösung suspendiert und unter Rühren abkühlen gelassen. Die klare Lösung wird im Vakuum zur Trockne eingedampft.

5

EP 0 445 158 B1

**Beispiel 7**

| | |
|---|---|
| Oxipurinol-Natrium-Monohydrat | 10,0 g |
| Destilliertes Wasser | 50,0 g |
| Polyvinylpyrrolidon/Polyvinylacetat-Mischpolymerisat (Kollidon® VA 64) | 5,0 g |
| Polyethylenglykol (Mittl. Mol.-Gewicht 10000) | 5,0 g |
| Mikrokristalline Cellulose (Avicel® PH 102) | 20,0 g |

Oxipurinol wird in Wasser bei 70°C gelöst. Wach Zugabe von Kollidon® und Polyethylenglykol wird in die klare Lösung die mikrokristalline Cellulose eingerührt. Die Suspension wird im Vakuum zur Trockne eingedampft.

**Beispiel 8**

| | |
|---|---|
| Oxipurinol-Natrium-Monohydrat | 5,0 g |
| Destilliertes Wasser | 250,0 g |
| Polyvinylpyrrolidon/Polyvinylacetat-Mischpolymerisat (Kollidon® VA 64) | 2,5 g |
| Polyethylenglykol (Mittl. Mol.-Gewicht 10000) | 2,5 g |

Die Herstellung der Feststoffdispersion erfolgt analog Beispiel 3.

| | |
|---|---|
| Feststoffdispersion | 3,00 g |
| Quervernetztes Polyvinylpyrrolidon (Polyplasdone® XL) | 1,50 g |
| Stearinsäure | 0,06 g |
| Hochdisperse Kieselsäure (Aerosil® 200) | 0,09 g |
| | 4,65 g |

Die Oxipurinol-haltige Feststoffdispersion wurde mit den angegebenen Hilfsstoffen vermischt und zu Tabletten mit einem Gehalt von 150 mg Wirkstoff verpreßt. 2 Tabletten, entsprechend 300 mg Oxipurinol-Natrium-Monohydrat, wurden auf Freisetzung des Wirkstoffes geprüft.

Die erfindungsgemäßen Formen wurden im Vergleich zu feingemörsertem, kristallinem Oxipurinol-Natrium-Monohydrat auf ihre Löslichkeit bzw. Lösungsgeschwindigkeit geprüft.

6

Prüfmodell:

| | |
|---|---|
| Dissolution-Tester: | United States Pharmakopöe (USP XXI) |
| Prüfmedium: | 0,1 N-Salzsäure |
| Prüfvolumen: | 1000 ml |
| Prüftemperatur: | 37°C |
| Umdrehungsgeschwindigkeit: | 90 U/min. |

Die Messungen erfolgten spektralphotometrisch bei 245 nm. Die Meßergebnisse sind aus der nachfolgenden Tabelle 1 ersichtlich.

**Tabelle 1:**

Gelöste Menge Wirkstoff in mg/Liter (kumulierte Werte) aus:

| | Vergleich | Bsp. 1 1. Versuch | Bsp. 1 2. Versuch | Bsp. 1 3. Versuch | Bsp. 4 | Bsp. 7 | Bsp. 8 |
|---|---|---|---|---|---|---|---|
| 5 Min. | 43 | 228 | 434 | 360 | 350 | 357 | 255 |
| 10 Min. | 62 | 245 | 429 | 380 | 384 | 388 | 267 |
| 20 Min. | 88 | 262 | - | - | 417 | 409 | 278 |
| 30 Min. | 115 | 266 | 406 | 377 | 413 | 411 | 285 |
| 45 Min. | 126 | - | 384 | - | 402 | 409 | 285 |
| 60 Min. | 142 | 270 | - | 381 | 407 | 411 | 289 |
| 180 Min | 207 | - | 340 | 374 | 404 | 393 | 287 |
| 24 Std. | 280 | 276 | 246 | - | - | - | - |
| 48 Std. | 263 | - | - | - | - | - | - |

Die erfindungsgemäßen Zubereitungen setzen demnach den darin enthaltenen Wirkstoff gegenüber dem Vergleich bereits nach einer Meßzeit von 5 Minuten beschleunigt frei, so daß sich die Lösungsgeschwindigkeit beispielhaft von etwa 43 mg/5 min. auf etwa 430 mg/5 min. steigern läßt, entsprechend hoch ist die terminale Löslichkeit, da der Wirkstoff nicht sofort wieder rekristallisiert, sondern im gelösten Zustand während seiner Verweilzeit im Magen und oberen Verdauungstrakt ausreichend lange zur Resorption zur Verfügung steht.

Darüber hinaus belegen die Ergebnisse aus Tabelle 2, daß die erfindungsgemäßen Zubereitungen auch in dem beanspruchten pH-Wert-Bereich von etwa 1,0 bis 7,5 eine der kristallinen Form überlegenen Löslich-

8

keit aufweisen.

Tabelle 2:

Löslichkeit gemäß Bsp. 1 (3. Versuch) in Abhängigkeit vom pH-Wert und der Zeit im Vergleich zu kristallinem Wirkstoff (Werte in mg/Liter)

| Zeit \ pH-Wert | pH = 1.5 | | pH = 3.0 | | pH = 4.5 | | pH = 6.5 | |
|---|---|---|---|---|---|---|---|---|
| | krist. | Feststoff-dispersion | krist. | Feststoff-dispersion | krist. | Feststoff-dispersion | krist. | Feststoff-dispersion |
| 5 Minuten | 43 | 360 | 81 | 382 | 85 | 435 | 243 | 502 |
| 10 Minuten | 62 | 380 | 110 | 402 | 110 | 431 | 261 | 528 |
| 60 Minuten | 143 | 381 | 222 | 398 | 243 | 423 | 290 | 562 |

**Patentansprüche**

1. Zubereitungen des Oxipurinols und/oder seiner Alkali- oder Erdalkalisalze, dadurch gekennzeichnet, daß sie den Wirkstoff in nicht kristalliner Form in Form einer sogenannten "festen Dispersion" zusammen mit pharmakologisch unbedenklichen Hilfsstoffen im Verhältnis 1:0,2 bis 1:10, vorzugsweise im Verhältnis 1:1 bis 1:4 enthalten.

2. Zubereitung gemäß Anspruch 1, dadurch gekennzeichnet, daß das darin enthaltene Oxipurinol eine Lösungsgeschwindigkeit bei einem pH-Wert zwischen 1,0 und 7,5 von mehr als 100 mg/l pro 5 Minuten besitzt.

3. Zubereitung gemäß Anspruch 1 oder 2, dadurch gekennzeichnet, daß die terminale (vorübergehende) Löslichkeit des darin enthaltenen Oxipurinols über der Sättigungskonzentration liegt und länger als eine Stunde aufrechterhalten wird.

4. Zubereitung gemäß einem der Ansprüche 1 bis 3, dadurch gekennzeichnet, daß sie weitere Wirkstoffe enthalten oder mit diesen vermischt vorliegen.

5. Verfahren zur Herstellung von Zubereitungen des Oxipurinols und/oder seiner Alkali- oder Erdalkalisalze in nicht kristalliner Form, dadurch gekennzeichnet, daß der Wirkstoff mit pharmakologisch unbedenklichen Hilfsstoffen im Verhältnis 1:0,2 bis 1:10, vorzugsweise im Verhältnis 1:1 bis 1:4, gegebenenfalls unter Zusatz eines Lösungsmittels gelöst wird oder in einer Hilfsstoffschmelze aufgelöst wird und die so erhaltenen Schmelzen oder Lösungen unter Vermeidung von Rekristallisationen abgekühlt und/ oder eingetrocknet werden.

6. Verfahren gemäß Anspruch 5, dadurch gekennzeichnet, daß als Lösungsmittel Wasser verwendet wird.

7. Verwendung von Zubereitungen gemäß einem der Ansprüche 1 bis 4 zur Herstellung von enteral applizierbaren Arzneimitteln.

8. Verwendung gemäß Anspruch 7 zur Herstellung von Arzneimitteln zur Behandlung der Hyperurikämie und der Gicht.


## Claims

1. Preparations of oxipurinol and/or its alkali metal or alkaline earth metal salts, characterized in that they contain the active ingredient in a non-crystalline form in the form of a so-called "solid dispersion" in combination with pharmacologically acceptable adjuvant in a ratio of from 1:0.2 to 1:10, and preferably in a ratio of from 1:1 to 1:4.

2. A preparation according to claim 1, characterized in that the oxipurinol contained therein has a dissolution rate at a pH value between 1.0 and 7.5 of more than 100 mg/l per 5 minutes.

3. A preparation according to claim 1 or 2, characterized in that the terminal (temporary) solubility of the oxipurinol contained therein is above the saturation concentration and is maintained for more than one hour.

4. A preparation according to anyone of claims 1 to 3, characterized in that it contains further active ingredients or is present in admixture with same.

5. A process for manufacturing preparations of oxipurinol and/or its alkali metal or alkaline earth metal salts in a non-crystalline form, characterized in that thw active ingredient is dissolved in combination with pharmacologically acceptable adjuvant in a ratio of from 1:0.2 to 1:10, and preferably in a ratio of from 1:1 to 1:4, optionally with the addition of a solvent, or is dissolved in a molten adjuvant, and the molten compositions or solutions obtained thereby are allowed to cool and/or dry up while recrystallisations are avoided.

6. The process according to claim 5, characterized in that water is used as the solvent.

7. Use of preparations according to anyone of claims 1 to 4 for the manufacture of medicaments for an enteral application.

8. The use according to claim 7 for the manufacture of medicaments for the treatments of hyperuricaemia and of gout.

**Revendications**

1. Préparations à base d'oxypurinol ou de ses sels alcalins ou alcalino-terreux, caractérisées en ce qu'elles contiennent la matière active à l'état non cristallin sous forme de ce qu'on appelle une "dispersion solide" avec des matières auxiliaires pharmacologiquement acceptables dans le rapport 1:0,2 à 1:10, de préférence 1:1 à 1:4.

2. Préparations selon la revendication 1, caractérisées en ce que l'oxypurinol qu'elles contiennent a, dans le cas d'une valeur de pH, entre 1,0 et 7,5, une vitesse de dissolution supérieure à 100 mg par litre, en 5 minutes.

3. Préparations selon l'une des revendications 1 ou 2, caractérisées en ce que la solubilité terminale (transitoire) de l'oxypurinol qu'elles contiennent se trouve au-delà de la concentration de saturation et se maintient pendant plus d'une heure.

4. Préparations selon l'une des revendications 1 à 3, caractérisées en ce qu'elles contiennent d'autres matières actives ou bien sont mélangées avec elles.

5. Procédé pour l'obtention de préparation à base d'oxypurinol et/ou de ses sels alcalins ou alcalino-terreux à l'état non cristallin, caractérisé en ce qu'on dissout la matière active avec des substances auxiliaires pharmacologiquement acceptables dans le rapport 1:0,2 à 1:10 de préférence 1:1 à 1:4, éventuellement avec addition d'un solvant ou on le dissout dans une matière auxiliaire fondue, et les solutions ou matières fondues ainsi obtenues sont refroidies ou solidifiées tout en évitant des recristallisations.

6. Procédé selon la revendication 5, caractérisé en ce qu'on utilise de l'eau comme solvant.

7. Utilisation de préparations selon l'une quelconque des revendications 1 à 4 pour l'obtention de médicaments à applications entérales.

8. Utilisation selon la revendication 7, pour l'obtention de médicaments pour le traitement de l'hyperuricémie et de l'arthrite.